(19) Europäisches Patentamt
European Patent Office
Office européen des brevets

(11) EP 1 414 830 B1

(12) EUROPÄISCHE PATENTSCHRIFT

(45) Veröffentlichungstag und Bekanntmachung des Hinweises auf die Patenterteilung:
**18.10.2006 Patentblatt 2006/42**

(51) Int Cl.:
*C07F 5/06* (2006.01) *A61K 31/28* (2006.01)
*A61P 35/00* (2006.01)

(21) Anmeldenummer: **02767331.8**

(22) Anmeldetag: **06.08.2002**

(86) Internationale Anmeldenummer:
**PCT/EP2002/008770**

(87) Internationale Veröffentlichungsnummer:
**WO 2003/016318 (27.02.2003 Gazette 2003/09)**

# (54) TUMORHEMMENDE LANTHANVERBINDUNGEN

TUMOUR INHIBITING LANTHANE COMPOUNDS

COMPOSES DE LANTHANE INHIBITEURS DE TUMEURS

(84) Benannte Vertragsstaaten:
**AT BE BG CH CY CZ DE DK EE ES FI FR GB GR IE IT LI LU MC NL PT SE SK TR**

(30) Priorität: **06.08.2001 DE 10138538**

(43) Veröffentlichungstag der Anmeldung:
**06.05.2004 Patentblatt 2004/19**

(73) Patentinhaber: **Faustus Forschungs Cie. Translational Cancer Research GmbH**
**04109 Leipzig (DE)**

(72) Erfinder: **KEPPLER, Bernhard**
**68766 Hockenheim (DE)**

(74) Vertreter: **Grünecker, Kinkeldey, Stockmair & Schwanhäusser Anwaltssozietät**
**Maximilianstrasse 58**
**80538 München (DE)**

(56) Entgegenhaltungen:

- **CHEMICAL ABSTRACTS, vol. 77, no. 26, 25. Dezember 1972 (1972-12-25) Columbus, Ohio, US; abstract no. 172097, FOSTER, RUSSELL J. ET AL: "Complexes of 1,8-naphthyridines. X. Complexes of 1,8-naphthyridine with rare earth nitrates" XP002219334 & INORG. CHIM. ACTA (1972), 6(3), 371-5 , 1972,**
- **CHEMICAL ABSTRACTS, vol. 74, no. 6, 8. Februar 1971 (1971-02-08) Columbus, Ohio, US; abstract no. 27594, CHARPENTIER, LINDA J. ET AL: "Rare earths. LXXXIV. Preparation and properties of 1,2-propanediamine complexes" XP002219335 & J. INORG. NUCL. CHEM. (1970), 32(11), 3575-84 , 1970,**
- **ROESKY, PETER W.: "Aminotroponimines as ligands for yttrium and lanthanide complexes" CHEMISCHE BERICHTE/RECUEIL (1997), 130(7), 859-862 , 1997, XP002219333**
- **GOLUB A.M. ZH. OBSCH. KHIM. Bd. 39, Nr. 6, 1969, Seiten 1382 - 1387**
- **PATIL B.K. CURR. SCI. Bd. 45, Nr. 19, 1976, Seiten 686 - 688**
- **EREMIN Y.G. ZH. NEORG. KHIM. Bd. 21, Nr. 2, 1976, Seiten 387 - 394**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 414 830 B1

## Beschreibung

Die vorliegende Erfindung betrifft Lanthanverbindungen sowie deren Verwendung als Arzneimittel zur Prophylaxe und/oder Behandlung von Krebserkrankungen.

Komplexe von 1,8-Naphthyridin mit Nitraten seltener Erden werden offenbart in Chemical Abstracts, Bd. 77, Abstract No. 172097.

1,2-Propandiamin-Komplexe von Lanthan werden beschrieben in Chemical Abstracts, Bd. 74, Abstract No. 27594.

Aminotroponimine als Liganden von Lanthan-Komplexen werden offenbart in Chem. Ber., Recueil (1997), 130 (7), 589-862.

Tumorhemmende Lanthanverbindungen werden beschrieben in Anti-Cancer Drug Design (2000), 15, 405-11. Weitere Lanthanverbindungen werden beschrieben in : Zn. Obsch. Khim., 39(6), 1969, 1382-7 Curr. Sci., 1976, 45(19), 686-8 Zn. Neorg. Khim., 1976, 21(2) 387-94.

Aufgabe der vorliegenden Erfindung ist es, eine Verbindung zur Verfügung zu stellen, die eine hohe Wirksamkeit zur Behandlung von Krebserkrankungen aufweist.

Diese Aufgabe wird gelöst durch eine Verbindung der allgemeinen Formel (I)

$$R^{i\oplus}\,\frac{i}{n}\,Y^{n\ominus} \qquad \text{(I),}$$

worin R eine Gruppe der allgemeinen Formel (A) ist

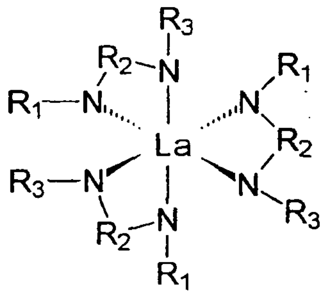

(A)

worin

$R_1$ und $R_3$ unabhängig voneinander ausgewählt sind aus der Gruppe, bestehend aus $C_1$ - $C_{10}$-Alkyl, $C_3$ - $C_6$-Cycloalkyl, $C_3$-$C_6$-Cycloalkenyl, $C_2$ - $C_{10}$-Alkenyl, $C_6$ - $C_{14}$-Aryl, und einem Heterocyclus, die jeweils substituiert oder unsubstituiert sein können, und Wasserstoff;

$R_2$ $C_1$ - $C_6$ -Alkylen, $C_3$ - $C_6$-Cycloalkylen, $C_3$ - $C_6$-Cycloalkenylen, $C_2$ - $C_6$ -Alkenylen, $C_6$ - $C_{14}$-Arylen oder ein Heterocyclus, die jeweils substituiert oder unsubstituiert sein können, ist;

und $R_1$ und $R_2$, und/oder $R_2$ und $R_3$ einen Heterocyclus bilden können, der gegebenenfalls weitere Stickstoffatome enthalten kann;

Y ein physiologisch verträgliches Anion ist; und

i und n unabhängig voneinander eine natürliche Zahl $\geq 1$ sind, und

physiologisch verträglichen Additionssalzen,

mit der Maßgabe, dass R nicht

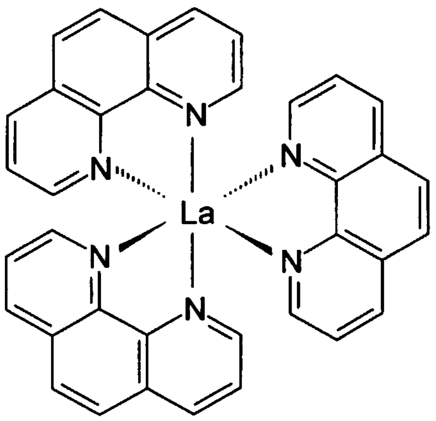

,

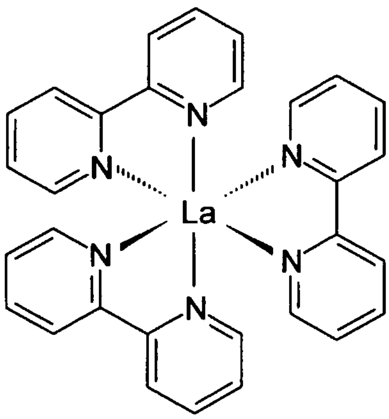

oder

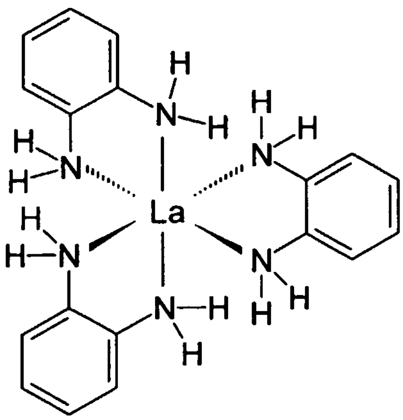

ist.

In einer bevorzugten Ausführungsform sind $R_1$ und/oder $R_3$ $C_1$-$C_5$ Alkyl, insbesondere Methyl, Ethyl oder Propyl. Ferner sind $R_1$ und/oder $R_3$ bevorzugt Cyclobutyl, Cyclopropyl, Cyclobutenyl oder Cyclopropenyl und insbesondere Cyclopentyl, Cyclohexyl, Cyclopentenyl oder Cyclohexenyl, oder $C_2$-$C_5$-Alkenyl, insbesondere Ethenyl, Propenyl oder Butenyl. Weiterhin können $R_1$ und/oder $R_3$ Benzyl oder Pyridyl sein.

$R_2$ ist bevorzugt $C_1$-$C_5$ Alkylen, insbesondere Methylen, Ethylen oder Propylen. Ferner ist $R_2$ bevorzugt Cyclobutylen, Cyclopropylen, Cyclopentylen, Cyclohexylen, Cyclopentenylen oder Cyclohexenylen oder $C_2$-$C_5$-Alkenylen, insbesondere Ethenylen, Propenylen oder Butenylen. Weiterhin kann $R_2$ Benzylen oder Pyridylen sein.

$R_1$, $R_2$ und/oder $R_3$ können durch Hydroxyl, Amino, $-SO_3H$, Halogen, $C_1$-$C_4$-Alkyl, $C_2$-$C_4$-Alkenyl, $C_3$-$C_6$-Cycloalkyl, $C_3$-$C_6$-Cycloalkenyl, $C_1$-$C_6$-Aryl, $C_1$-$C_4$-Alkoxy, $C_1$-$C_4$-Alkoxy-$C_1$-$C_4$-alkylen, $C_1$-$C_4$-Alkylmercapto, $C_1$-$C_4$-Alkylmercapto-$C_1$-$C_4$-alkylen, Formyl, Carboxyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamino-$C_1$-$C_4$-alkylen, Di-$C_1$-$C_4$-alkylaminocarbonyl, Di-$C_1$-$C_4$-alkylaminocarbonyl-$C_1$-$C_4$-alkylen, vorzugsweise Halogen und insbesondere Methyl, Ethyl oder Propyl substituiert sein, insbesondere wenn $R_1$ und $R_2$, und/oder $R_2$ und $R_3$ einen Heterocyclus bilden.

In einer weiteren bevorzugten Ausführungsform ist "i" die Zahl 3 und/oder "n" die Zahl 1.

Weiterhin ist Y in der allgemeinen Formel (I) bevorzugt ein Metallhalogen, ein Halogen, ein Pseudohalogen, $HCO_3$ oder R'COO, worin R' $C_1$ - $C_6$ -Alkyl, $C_2$ - $C_6$ -Alkenyl oder Aryl, die jeweils substituiert oder unsubstituiert sein können. Insbesondere ist Y SCN.

Organische oder anorganische Additionssalze können mit folgenden Anionen gebildet werden:

Chlorid, Bromid, Phosphat, Carbonat, Nitrat, Perchlorat, Sulfat, Citrat, Lactat, Tartrat, Maleat, Fumarat, Mandelat, Benzoat, Ascorbat, Cinnamat, Glycollat, Methansulfonat, Formiat, Malonat, Naphthalin-2-sulfonat, Salicylat und/oder Acetat.

Als mögliche Kationen können $H^+$, Natrium- und/oder Kalium-Kationen verwendet werden.

Ferner wird die Aufgabe der vorliegenden Erfindung durch ein Arzneimittel gelöst, enthaltend eine Verbindung der allgemeinen Formel (I)

$$R^{i\oplus}\,\frac{i}{n}\,Y^{n\ominus} \qquad (I),$$

worin R eine Gruppe der allgemeinen Formel (A) ist

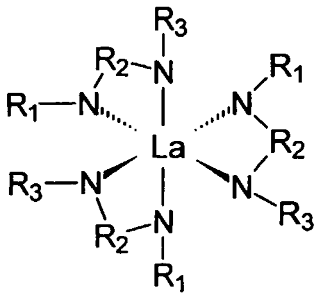

(A)

worin

$R_1$ und $R_3$ unabhängig voneinander ausgewählt sind aus der Gruppe bestehend aus $C_1$ - $C_{10}$ -Alkyl, $C_3$-$C_6$-Cycloalkyl, $C_3$-$C_6$-Cycloalkenyl, $C_2$-$C_{10}$-Alkenyl, $C_6$-$C_{14}$-Aryl, und einem Heterocyclus, die jeweils substituiert oder unsubstituiert sein können, und Wasserstoff;

$R_2$ $C_1$ - $C_6$ -Alkylen, $C_3$ - $C_6$-Cycloalkylen, $C_3$ - $C_6$-Cycloalkenylen, $C_2$ - $C_6$ -Alkenylen, $C_6$ - $C_{14}$-Arylen oder ein Heterocyclus, die jeweils substituiert oder unsubstituiert sein können, ist;

und $R_1$ und $R_2$, und/oder $R_2$ und $R_3$ einen Heterocyclus bilden können, der gegebenenfalls weitere Stickstoffatome enthalten kann;

Y ein physiologisch verträgliches Anion ist; und

i und n unabhängig voneinander eine natürliche Zahl $\geq 1$ sind, und

physiologisch verträglichen Additionssalzen.

Für das Arzneimittel, enthaltend eine Verbindung der allgemeinen Formel (I) sind hinsichtlich der Gruppen $R_1$, $R_2$, $R_3$, Y, i und n dieselben Ausführungsformen bevorzugt wie oben für die erfindungsgemäße Verbindung dargestellt.

In bevorzugten Ausführungsformen kann die Gruppe R der allgemeinen Formel (A) aus

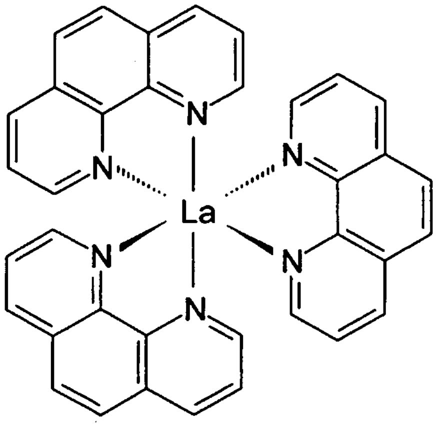

oder

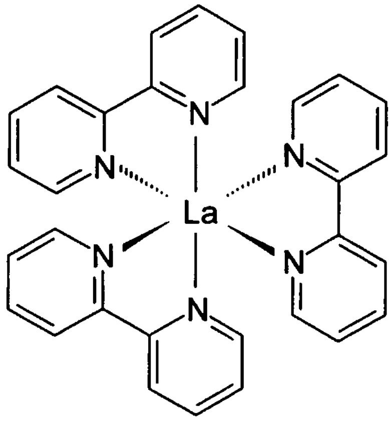

gewählt werden.

Die erfindungsgemäße Verbindung kann zur Prophylaxe und/oder Behandlung von Krebserkrankungen eingesetzt werden.

Die Aufgabe wird in einer weiteren Ausführungsform gelöst durch eine Verbindung der allgemeinen Formel (II)

$$R_b^{+}\, Y_b^{-} \qquad \text{(II)}$$

worin $R_b$ eine Gruppe der allgemeinen Formel (B) ist

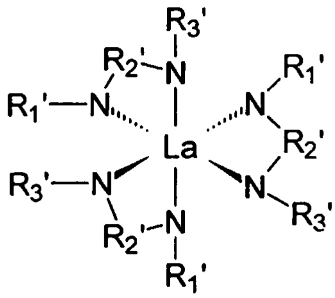

(B)

worin

$R_1$' und $R_3$' $C_1$ - $C_{10}$ -Alkyl, $C_3$ - $C_6$- Cycloalkyl, $C_2$ - $C_{10}$-Alkenyl, $C_6$ - $C_{14}$-Aryl, oder ein Heterocyclus, die jeweils substituiert oder unsubstituiert sein können, oder Wasserstoff ist;

$R_2$' $C_1$ - $C_6$ -Alkylen, $C_3$ - $C_6$ -Cycloalkylen, $C_2$ - $C_6$ -Alkenylen, $C_6$ - $C_{14}$-Arylen oder ein Heterocyclus, die jeweils substituiert oder unsubstituiert sein können, ist;

und $R_1$' und $R_2$', oder $R_2$' und $R_3$' einen Heterocyclus bilden können, der gegebenenfalls weitere Stickstoffatome enthalten kann;

und
$Y_b$ ein Metallhalogen, ein Halogen, ein Pseudohalogen, $HCO_3$ oder R'COO, worin R' $C_1$ - $C_6$ -Alkyl, $C_2$ - $C_6$ -Alkenyl oder Aryl, die jeweils substituiert oder unsubstituiert sein können, ist.

In bevorzugten Ausführungsformen kann die Gruppe $R_b$ der allgemeinen Formel (B) aus

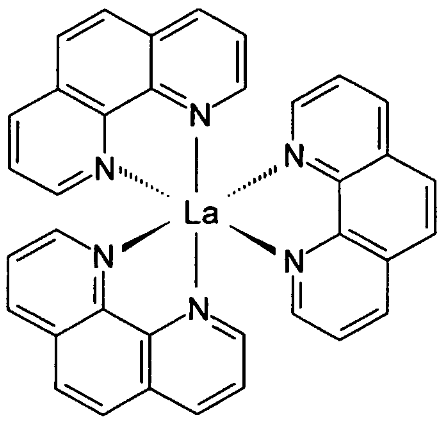
,
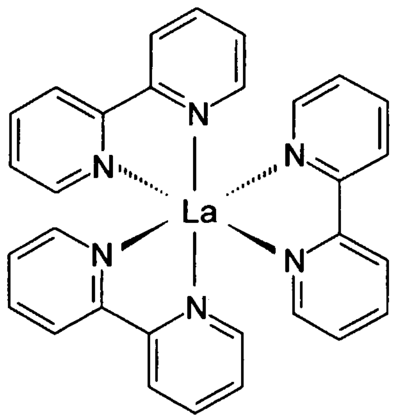

gewählt werden.

$R_1$' und $R_3$' sind bevorzugt $C_1$-$C_5$ Alkyl, insbesondere Methyl, Ethyl oder Propyl. Ferner sind $R_1$' und $R_3$' bevorzugt Cyclobutyl, Cyclopropyl oder $C_2$-$C_5$-Alkenyl, insbesondere Ethenyl, Propenyl oder Butenyl. Weiterhin können $R_1$' und $R_3$' Benzyl oder Pyridyl sein.

$R_1$' und $R_3$' können durch Methyl, Ethyl oder Propyl substituiert sein, insbesondere wenn $R_1$' und $R_2$', oder $R_2$' und $R_3$' einen Heterocyclus bilden.

$R_2$' ist bevorzugt $C_1$-$C_5$ Alkylen, insbesondere Methylen, Ethylen oder Propylen. Ferner ist $R_1$' und $R_3$' bevorzugt Cyclobutylen, Cyclopropylen oder $C_2$-$C_5$-Alkenylen, insbesondere Ethenylen, Propenylen oder Butenylen. Weiterhin kann $R_2$' Benzylen oder Pyridylen sein.

$R_2$' kann durch Methyl, Ethyl oder Propyl substituiert sein, insbesondere wenn $R_1$' und $R_2$' oder $R_2$' und $R_3$' einen Heterocyclus bilden.

Weiterhin ist $Y_b$ in der allgemeinen Formel (II) bevorzugt SCN.

Ferner wird die Aufgabe der vorliegenden Erfindung durch ein Arzneimittel gelöst, das die erfindungsgemäße Verbindung enthält. Die erfindungsgemäße Verbindung kann zur Prophylaxe und/oder Behandlung von Krebserkrankungen eingesetzt werden.

Im folgenden wird das Arzneimittel, enthaltend eine erfindungsgemäße Verbindung, genauer beschrieben.

Das erfindungsgemäße Arzneimittel wird vor allem intravenös, aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Das Arzneimittel wird nach an sich bekannten Verfahren hergestellt, wobei die erfindungsgemäße Verbindung als solche oder gegebenenfalls in Kombination mit geeigneten pharmazeutischen Trägerstoffen eingesetzt wird. Enthält das erfindungsgemäße Arzneimittel neben dem Wirkstoff pharmazeutische Trägerstoffe, beträgt der Wirkstoffgehalt dieser Mischung 0,1 bis 99,5, vorzugsweise 0,5 bis 95 Gew.-% der Gesamtmischung.

Das erfindungsgemäße Arzneimittel kann in jeder geeigneten Formulierung angewandt werden unter der Voraussetzung, dass die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit verstanden, die eine individuelle Menge des aktiven Bestandteils in Kombination mit einem pharmazeutischen Trägerstoff enthält und deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die erfindungsgemäßen Arzneimittel können, wenn sie in Einheitsdosen vorliegen und für Applikationen z.B. am Menschen bestimmt sind, etwa 0,1 bis 500 mg, bevorzugt 10 bis 200 mg und insbesondere 50 bis 150 mg Wirkstoff enthalten.

Im allgemeinen werden in der Humanmedizin der oder die Wirkstoffe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur

Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht. Bei einer oralen Behandlung können ähnliche Dosierungen zur Anwendung kommen.

Die therapeutische Verabreichung des erfindungsgemäßen Arzneimittels kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Es kann sich auch als zweckmäßig erweisen, die Arzneimittel nur einmalig oder im Abstand von mehreren Tagen zu verabreichen.

Die Festlegung der erforderlichen optimalen Dosierung und Applikationsart der Wirkstoffe kann durch jeden Fachmann aufgrund seines Fachwissens erfolgen.

Die erfindungsgemäßen Arzneimittel bestehen in der Regel aus den erfindungsgemäßen Verbindungen und nichttoxischen, pharmazeutisch verträglichen Arzneimittelträgern, die als Zumischung oder Verdünnungsmittel, beispielsweise in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten Dragees, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Laktose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption der Arzneimittelzubereitung im Gastrointestinaltrakt bewirkt, so dass z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuss-, oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die erfindungsgemäße Verbindung in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuss-, oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmittel enthalten.

Wässrige Lösungen können Konservierungsmittel, z.B. Methyl- oder Propylhydroxybenzoate; Verdickungsmittel; Geschmacksmittel; Süßungsmittel, z.B. Saccharose, Laktose, Natriumcyclamat, Dextrose, Invertzuckersirup, sowie Geschmacksmittel und Farbstoffe enthalten.

Zur parenteralen Anwendung der Arzneistoffe dienen steril injizierbare, wässrige Lösungen, isotonische Salzlösungen oder sonstige Lösungen.

Die folgenden Beispiele erläutern die Erfindung.

**Beispiel 1**

**Synthese von [Tris(1,10-phenantrolin)lanthan(III)]trithiocyanat**

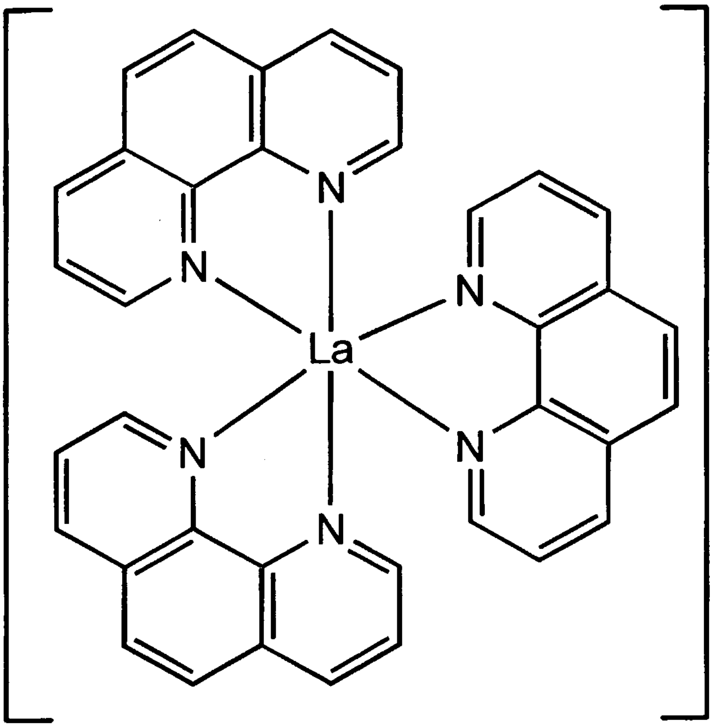

$(SCN)_3$

Die Herstellung von [Tris(1,10-phenantrolin)lanthan(III)]trithiocyanat erfolgt durch Vereinigung von Lanthantrichloridhexahydrat ($LaCl_3$ 6 $H_2O$) in 0,05 M ethanolischer Lösung mit Kaliumthiocyanat (KSCN) in 0,053 M ethanolischer Lösung in einem molaren Verhältnis von 1:4. Nach Abfiltrieren des entstehenden Kaliumchlorid-Niederschlags wird das Filtrat langsam unter Rühren zu einer 0,1 M ethanolischen 1,10-Phenatrolinmonohydrat-Lösung getropft. Das dabei entstehende feinkristalline Produkt wird filtriert, mehrmals mit Ethanol gewaschen und im Vakuum über Calciumsulfat getrocknet.

**Beispiel 2**

**Tumorhemmende Aktivität von [Tris(1,10-phenantrolin)lanthan(III)]trithiocyanat**

Im 48 h-Sulforhodamin B-Assay an über 50 humanen Tumorzellinien wurde eine gute Wirksamkeit mit folgenden Kennwerten festgestellt:

| | | |
|---|---|---|
| mittlere $GI_{50}$: | 1,29 μmol/l | 1,10 μg/ml |
| mittlere TGI: | 13,2 μmol/l | 11,3 μg/ml |
| mittlere $LC_{50}$: | 55,0 μmol/l | 46,9 μg/ml |

Überdurchschnittliche Aktivitäten wurden hierbei insbesondere an einigen Melanom- und Nierenzellkarzinom-Zellinien beobachtet.

Im Propidiumiodid-Assay an 13 humanen Tumorxenografts und 10 humanen Tumorzellinien ebenfalls eine gute tumorhemmende Aktivität mit folgenden Kenndaten:

| | | |
|---|---|---|
| mittlere $IC_{50}$: | 4,21 μmol/l | 3,60 μg/ml |
| mittlere $IC_{70}$: | 7,90 μmol/l | 6,74 μg/ml |
| mittlere $IC_{90}$: | 14,2 μmol/l | 12,1 μg/ml |

Dabei wurde eine Selektivität für Prostata- und kolorektales Karzinom beobachtet. Überdurchschnittliche Aktivitäten wurden weiters an zwei Mammakarzinom-Zellinien (MCF7, MDA468A) und einer kleinzelligen Bronchialkarzinom-Zellinie (DMS 114) sowie an je einem Melanom-, Ovarial-, Nierenzell- und nicht-kleinzelligen Bronchialkarzinom-Xenograft festgestellt.

## Patentansprüche

1. Verbindung der allgemeinen Formel (I)

$$R^{i\oplus}\frac{i}{n}Y^{n\ominus} \qquad (I),$$

worin R eine Gruppe der allgemeinen Formel (A) ist

(A) 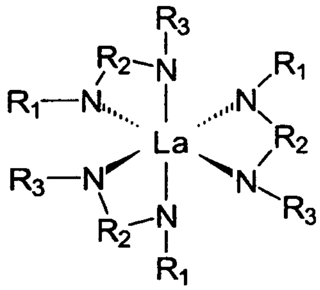

worin

R$_1$ und R$_3$ unabhängig voneinander ausgewählt sind aus der Gruppe bestehend aus $C_1$ - $C_{10}$ -Alkyl, $C_3$ - $C_6$-Cycloalkyl, $C_3$ - $C_6$-Cycloalkenyl, $C_2$- $C_{10}$-Alkenyl, $C_{6-}C_{14}$-Aryl, und einem Heterocyclus, die jeweils substituiert oder unsubstituiert sein können, und Wasserstoff;
$R_2$ $C_1$ - $C_6$ -Alkylen, $C_3$ - $C_6$-Cycloalkylen, $C_3$ - $C_6$-Cycloalkenylen, $C_2$ - $C_6$ - Alkenylen, $C_6$ - $C_{14}$-Arylen oder ein Heterocyclus, die jeweils substituiert oder unsubstituiert sein können, ist;
und $R_1$ und $R_2$, und/oder $R_2$ und $R_3$ einen Heterocyclus bilden können, der gegebenenfalls weitere Stickstoffatome enthalten kann;
Y ein physiologisch verträgliches Anion ist; und
i und n unabhängig voneinander eine natürliche Zahl ≥ 1 sind, und
physiologisch verträglichen Additionssalzen,
mit der Maßgabe, dass R nicht

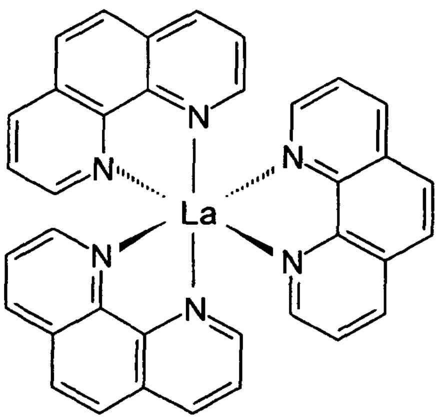

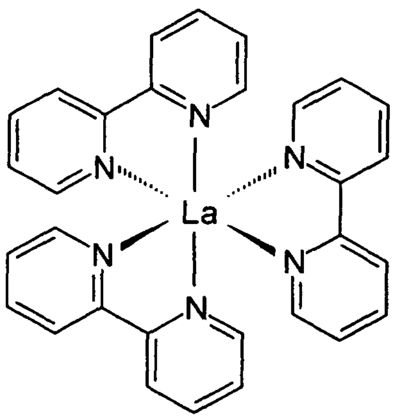

,

oder

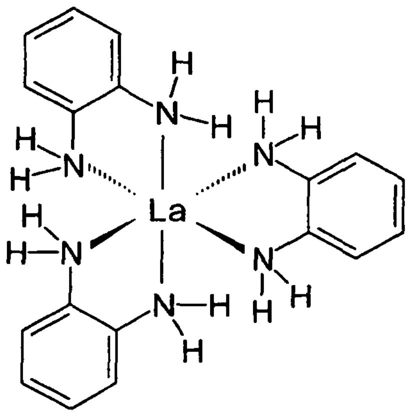

ist, dass R nicht

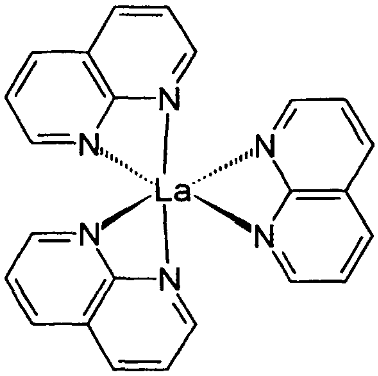

ist, falls Y $NO_3^-$ ist und dass R nicht

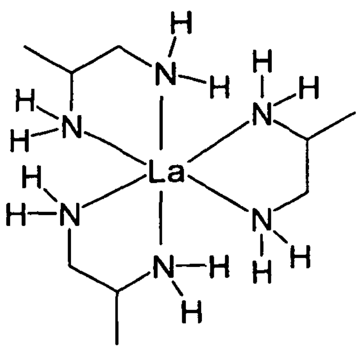

ist, falls Y $NO_3^-$, $ClO_4^-$ oder $Cl^-$ ist.

2. Verbindung nach Anspruch 1, wobei Y in der allgemeinen Formel (I) SCN ist.

3. Arzneimittel, enthaltend eine Verbindung der allgemeinen Formel (I)

$$R^{i\oplus}\,\frac{i}{n}\,Y^{n\ominus} \qquad (I),$$

worin R eine Gruppe der allgemeinen Formel (A) ist

(A) 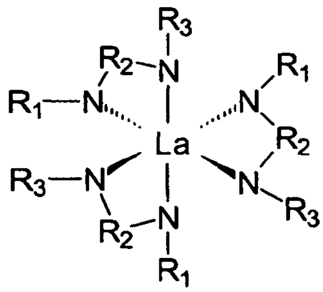

worin

$R_1$ und $R_3$ unabhängig voneinander ausgewählt sind aus der Gruppe bestehend aus $C_1$ - $C_{10}$ -Alkyl, $C_3$ - $C_6$-Cycloalkyl, $C_3$-$C_6$-Cycloalkenyl, $C_2$ - $C_{10}$-Alkenyl, $C_6$ - $C_{14}$-Aryl, und einem Heterocyclus, die jeweils substituiert oder unsubstituiert sein können, und Wasserstoff;
$R_2$ $C_1$ - $C_6$ -Alkylen, $C_3$ - $C_6$-Cycloalkylen, $C_3$ - $C_6$-Cycloalkenylen, $C_2$ - $C_6$-Alkenylen, $C_6$ - $C_{14}$-Arylen oder ein Heterocyclus, die jeweils substituiert oder unsubstituiert sein können, ist;
und $R_1$ und $R_2$, und/oder $R_2$ und $R_3$ einen Heterocyclus bilden können, der gegebenenfalls weitere Stickstoffatome enthalten kann;
Y ein physiologisch verträgliches Anion ist; und
i und n unabhängig voneinander eine natürliche Zahl $\geq 1$ sind, und
physiologisch verträglichen Additionssalzen.

**4.** Arzneimittel nach Anspruch 3, wobei die Gruppe R der Verbindung der allgemeinen Formel (I)

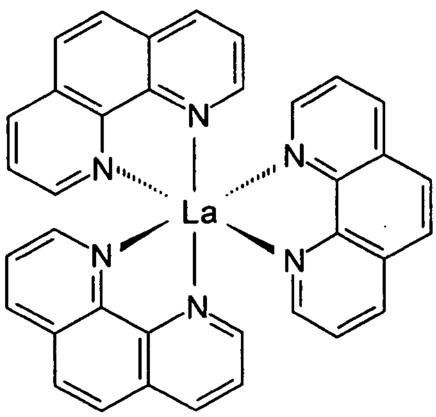

ist.

**5.** Arzneimittel nach Anspruch 3, wobei die Gruppe R der Verbindung der allgemeinen Formel (I)

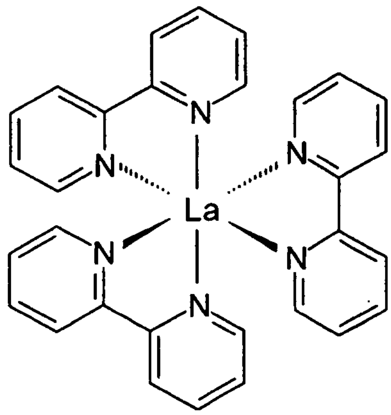

ist.

6. Verwendung einer Verbindung der allgemeinen Formel (I) nach einem der Ansprüche 1 oder 2 oder einer Verbindung der allgemeinen Formel (I), wie in einem der Ansprüche 3 bis 5 dargestellt, zur Herstellung eines Arzneimittels zur Prophylaxe und/oder Behandlung von Krebserkrankungen.

## Claims

1. Compound of the general formula (I)

$$R^{i\oplus}\frac{i}{n}\,Y^{n\ominus} \qquad \text{(I),}$$

where R is a group of the general formula (A):

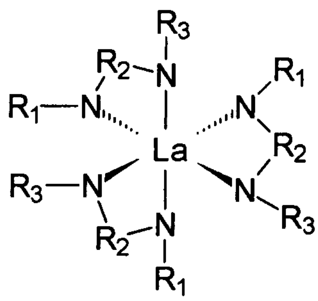

where

$R_1$ and $R_3$ are selected independently of one another from the group consisting of $C_1$-$C_{10}$-alkyl, $C_3$-$C_6$-cycloalkyl, $C_3$-$C_6$-cycloalkenyl, $C_2$-$C_{10}$-alkenyl, $C_6$-$C_{14}$-aryl and a heterocycle, which can in each case be substituted or unsubstituted, and hydrogen;
$R_2$ is $C_1$-$C_6$-alkylene, $C_3$-$C_6$-cycloalkylene, $C_3$-$C_6$-cycloalkenylene, $C_2$-$C_6$-alkenylene, $C_6$-$C_{14}$-arylene or a heterocycle, which can in each case be substituted or unsubstituted;
and $R_1$ and $R_2$ and/or $R_2$ and $R_3$ can form an heterocycle which, where applicable, can contain further nitrogen atoms;
Y is a physiologically compatible anion; and
i and n are independent of one another and are natural numbers ≥ 1, and
physiologically compatible addition salts,

provided that R is not:

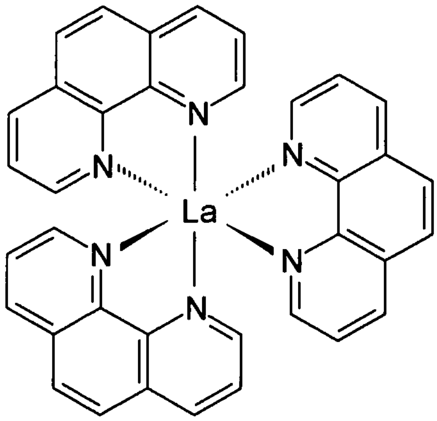

,

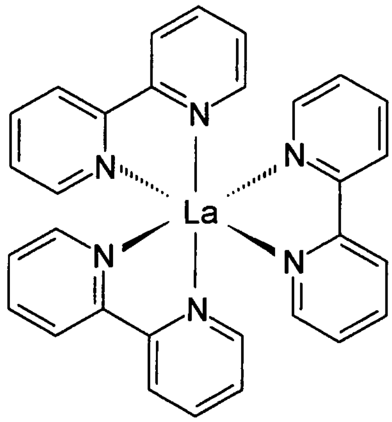

,

or

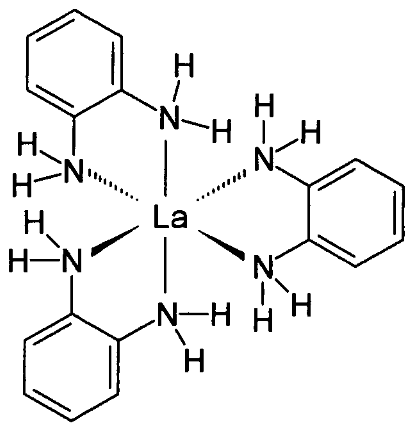

and that R is not

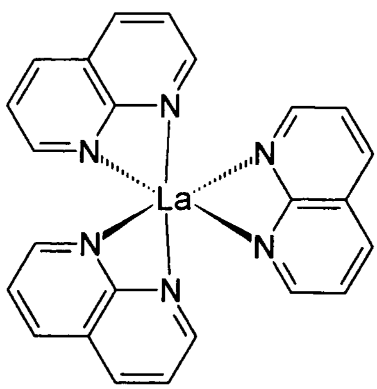

if Y is $NO_3^-$ and
that R is not

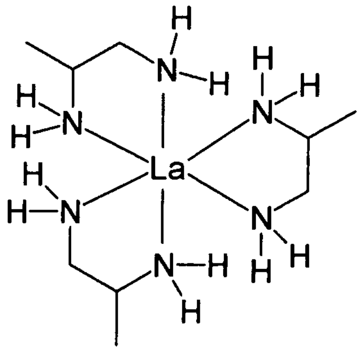

if Y is $NO_3^-$, $ClO_4^-$ or $Cl^-$.

**2.** Compound according to Claim 1, wherein Y in the general formula (I) is SCN.

**3.** Medicament, containing a compound of the general formula (I)

$$R^{i\oplus}\frac{i}{n}Y^{n\ominus} \qquad (I),$$

where R is a group of the general formula (A)

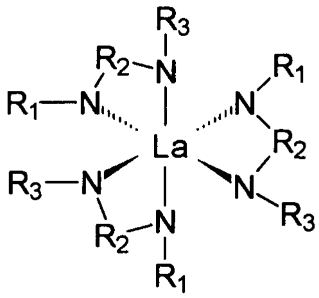

where

$R_1$ and $R_3$ are selected independently of one another from the group consisting of $C_{1-}C_{10}$-alkyl, $C_3$-$C_6$-cycloalkyl, $C_3$-$C_6$-cycloalkenyl, $C_2$-$C_{10}$-alkenyl, $C_6$-$C_{14}$-aryl and a heterocycle, which can in each case be substituted or unsubstituted, and hydrogen;
$R_2$ is $C_1$-$C_6$-alkylene, $C_3$-$C_6$-cycloalkylene, $C_3$-$C_6$-cycloalkenylene, $C_2$-$C_6$-alkenylene, $C_6$-$C_{14}$-arylene or a heterocycle, which can in each case be substituted or unsubstituted ;
and $R_1$ and $R_2$ and/or $R_2$ and $R_3$ can form an heterocycle which, where applicable, can contain further nitrogen atoms;
Y is a physiologically compatible anion; and
i and n are independent of one another and are natural numbers ≥ 1, and
physiologically compatible addition salts.

**4.** Medicament according to Claim 3, whereby the group R of the compound of the general formula (I) is:

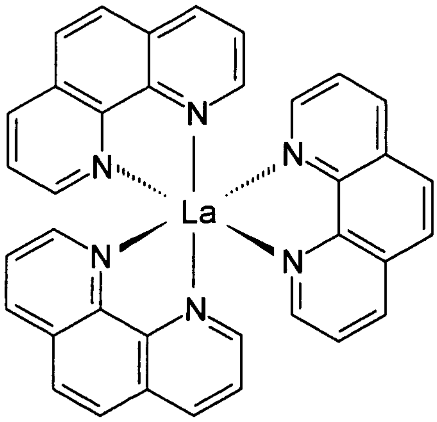

**5.** Medicament according to Claim 3, whereby the group R of the compound of the general formula (I) is:

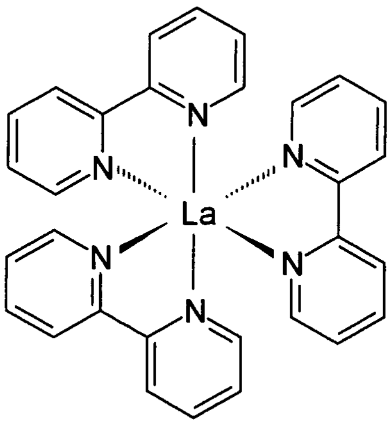

**6.** Use of a compound of the general formula (I) according to one of Claims 1 or 2 or a compound of the general formula (I) as presented in one of the Claims 3 to 5 for the preparation of a medicament for the prophylaxis and/or treatment of cancer diseases.

## Revendications

**1.** Composé de formule générale (I)

$$R^{i\oplus} \ \frac{i}{n} Y^{n\ominus} \qquad (I)$$

dans laquelle R est un groupe de formule générale (A),

(A) 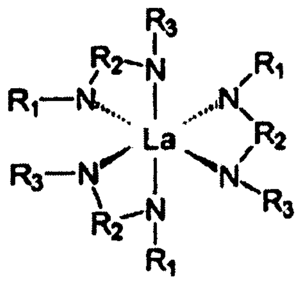

dans laquelle

$R_1$ et $R_3$ sont choisis, indépendamment l'un de l'autre, dans l'ensemble constitué par alkyle en $C_1$ à $C_{10}$, cycloalkyle en $C_3$ à $C_6$, cycloalcényle en $C_3$ à $C_6$, alcényle en $C_2$ à $C_{10}$, aryle en $C_6$ à $C_{14}$, et un hétérocycle, qui peuvent chacun être substitué ou non substitué, et l'hydrogène ;
$R_2$ est alkylène en $C_1$ à $C_6$, cycloalkylène en $C_3$ à $C_6$, cycloalcénylène en $C_3$ à $C_6$, alcénylène en $C_2$ à $C_6$, arylène en $C_6$ à $C_{14}$ ou un hétérocycle, qui peuvent chacun être substitué ou non substitué ;
et $R_1$ et $R_2$, et/ou $R_2$ et $R_3$, peuvent former un hétérocycle, qui éventuellement peut contenir d'autres atomes d'azote ;
Y est un anion compatible d'un point de vue physiologique ; et
i et n représentent indépendamment l'un de l'autre un nombre entier ≥ 1, et
sels d'addition compatibles d'un point de vue physiologique,
avec la réserve que R n'est pas

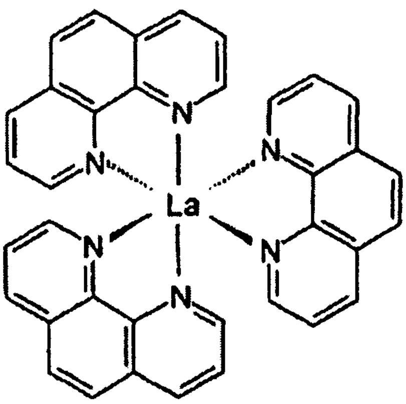

,

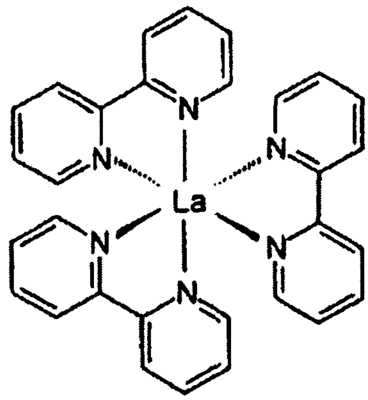

ou

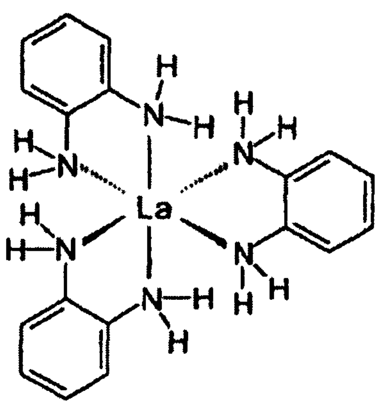

que R n'est pas

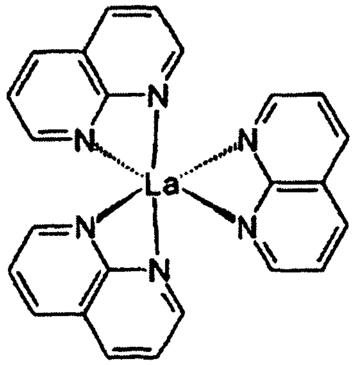

quand Y est $NO_3^-$,
et que R n'est pas

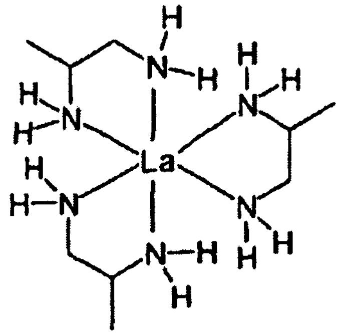

quand Y est $NO_3^-$, $ClO_4^-$ ou $Cl^-$.

**2.** Composé selon la revendication 1, dans lequel Y, dans la formule générale (I), est SCN.

**3.** Médicament contenant un Composé de formule générale (I)

$$R^{i\oplus} \frac{i}{n} Y^{n\ominus} \qquad (I)$$

dans laquelle R est un groupe de formule générale (A),

(A) 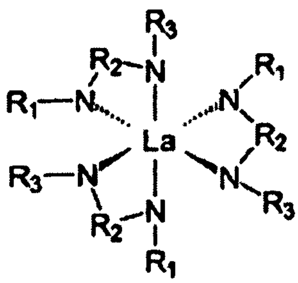

dans laquelle

$R_1$ et $R_3$ sont choisis, indépendamment l'un de l'autre, dans l'ensemble constitué par alkyle en $C_1$ à $C_{10}$, cycloalkyle en $C_3$ à $C_6$, cycloalcényle en $C_3$ à $C_6$, alcényle en $C_2$ à $C_{10}$, aryle en $C_6$ à $C_{14}$, et un hétérocycle, qui peuvent chacun être substitué ou non substitué, et l'hydrogène ;
$R_2$ est alkylène en $C_1$ à $C_6$, cycloalkylène en $C_3$ à $C_6$, cycloalcénylène en $C_3$ à $C_6$, alcénylène en $C_2$ à $C_6$, arylène en $C_6$ à $C_{14}$ ou un hétérocycle, qui peuvent chacun être substitué ou non substitué ;
et $R_1$ et $R_2$, et/ou $R_2$ et $R_3$, peuvent former un hétérocycle, qui éventuellement peut contenir d'autres atomes d'azote ;
Y est un anion compatible d'un point de vue physiologique ; et
i et n représentent indépendamment l'un de l'autre, un nombre entier ≥ 1, et
sels d'addition compatibles d'un point de vue physiologique.

**4.** Médicament selon la revendication 3, dans lequel le groupe R du composé de formule générale (I) est

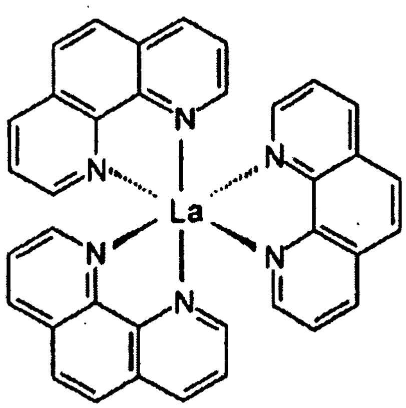

**5.** Médicament selon la revendication 3, dans lequel le groupe R du composé de formule générale (I) est

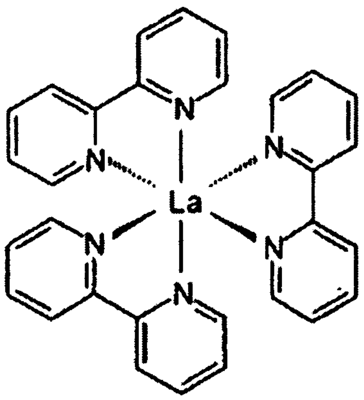

**6.** Utilisation d'un composé de formule générale (I) selon l'une des revendications 1 et 2 ou d'un composé de formule générale (I) tel que représenté dans l'une des revendications 3 à 5, pour la préparation d'un médicament destiné à la prophylaxie et/ou au traitement de maladies cancéreuses.